# DEMANDE DE BREVET EUROPEEN

(11) **EP 2 939 742 A1**
(43) Date de publication de la demande: **04.11.2015**
(21) Numéro de dépôt: 15305334.3
(22) Date de dépôt: 04.03.2015
(51) Int. Cl.: B01J 31/02, B01J 31/18, B01J 31/22, C07C 2/32

(54) **Nouvelle composition catalytique à base de nickel et son utilisation dans un procédé d'oligomérisation des oléfines**

(30) Priorité: 28.04.2014 FR 1453816
(71) Demandeur: IFP Énergies nouvelles, 92852 Rueil-Malmaison Cedex (FR); Universiteit van Amsterdam, 1000 GG Amsterdam (NL)
(72) Inventeur: Boulens, Pierre, 69006 Lyon (FR); Breuil, Pierre-Alain, 69006 LYON (FR); Reek, Joost, NL-3817 BK Amersfoort (NL); Olivier-Bourbigou, Helene, 69230 SAINT GENIS-LAVAL (FR)
(74) Mandataire: Ruis, Alain

(57) **Abrégé**

L'invention décrit une composition à base de nickel. L'invention concerne également l'utilisation de ladite composition comme composition catalytique dans un procédé d'oligomérisation des oléfines.

## Description

La présente invention concerne une nouvelle composition à base de nickel. L'invention concerne également l'utilisation de ladite composition comme catalyseur de réactions de transformations chimiques.

### Art antérieur:

Il est connu de préparer des compositions catalytiques à base de métaux de transition pour leur application dans divers domaines de la chimie, notamment dans le domaine des transformations catalytiques tels que l'hydroformylation, l'hydrogénation, le couplage croisé, l'oligomérisation des oléfines...

La préparation de telles compositions catalytiques passe par le choix du métal et des ligands adaptés. Parmi ces ligands, les ligands bidentes représentent une classe importante de ligands utilisés dans la préparation de compositions catalytiques à base de métaux de transition pour divers types de transformations catalytiques.

Le document EP 2 220 099 B1 décrit un système de complexes de coordination comprenant des ligands multidentes ayant la formule: R₁-SO₂-NH-P(XR₂)₂; ou R₁-SO₂-N=PH(XR₂)₂, ou R₁-SO(OH)=NP(XR₂)₂, dans lesquels X est indépendamment O, S, NH, ou une liaison; dans lequel R₁ et R₂ sont indépendamment choisis parmi un groupe alkyle substitué ou non et un groupe aryle, dans lequel au moins un équivalent de ligand est complexé à un équivalent d'un métal choisi parmi le rhodium, l'iridium, le platine, le palladium et les lanthanides. EP 2 220 099 B1 indique que le système de complexe de coordination peut être utilisé en tant que catalyseur pour l'hydroformylation, l'hydrogénation, l'hydrogénation, la polymérisation, l'isomérisation...

La demanderesse dans ses recherches a mis au point une nouvelle composition à base de nickel. Il a été constaté de manière surprenante que de telles compositions présentent des propriétés catalytiques intéressantes. En particulier, ces compositions présentent une bonne activité catalytique dans l'oligomerisation des oléfines, plus précisément dans la dimérisation de l'éthylène en butène-1.

Un objectif de l'invention est de fournir une nouvelle composition à base de nickel. Un autre objectif de l'invention est de proposer un nouveau système catalytique comprenant ladite composition pour des réactions de transformations chimiques, en particulier pour l'oligomerisation des oléfines.

### Description détaillée de l'invention

### Composition selon l'invention

La composition catalytique selon l'invention comprend:
- au moins un précurseur de nickel de degré d'oxydation (0) ou (+II),
- au moins un ligand répondant à la formule 1 a), 1 b) ou 1 c) 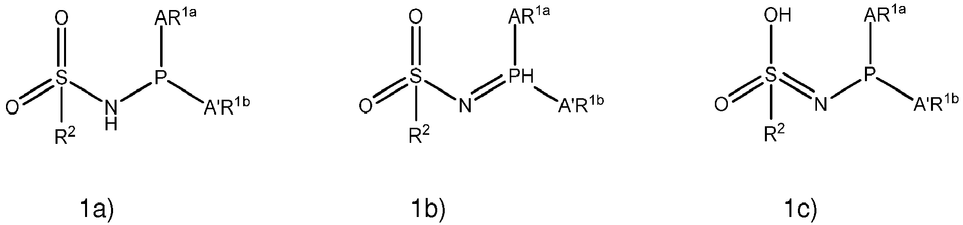
dans laquelle
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments, avec la condition que lorsque qu'un précurseur de nickel de degré d'oxydation (+II) est utilisé dans la composition, celui-ci est utilisé en présence d'un agent réducteur ou en présence d'une base de Brönsted.

Au sens de la présente invention, on entend par « alkyle » une chaîne hydrocarbonée linéaire ou ramifiée ayant de 1 à 15 atomes de carbone, de préférence de 1 à 10. Des groupes alkyles préférés sont avantageusement choisis parmi les groupes méthyle, éthyle, propyle, isopropyle, butyle, isobutyle et tertiobutyle. Ces groupements alkyles peuvent être substitués par des hétéroéléments ou des groupes contenant des hétéroéléments tels qu'un halogénure, un alcoxy. On entend par un substituant « alcoxy », un groupe alkyl-O- dans lequel le terme alkyle a la signification donnée ci-dessus. Des exemples préférés de substituants alcoxy sont les groupes méthoxy ou éthoxy.

Par « alkyle cyclique », on entend on entend un groupe hydrocarboné monocyclique ayant un nombre de carbone supérieur à 3, de préférence entre 4 et 24, de manière plus préférée entre 6 et 12, de préférence un groupe cyclopentyle, cyclohexyle, cyclooctyle ou cyclododécyle, ou polycyclique (bi- ou tricyclique) ayant un nombre de carbone supérieur à 3, de préférence entre 4 et 18, tels que par exemple les groupes adamantyle ou norbornyle.

Par « alkyle insaturé linéaire » ou « alkyle insaturé cyclique », on entend un alkyle linéaire ou cyclique possédant au moins une insaturation, le terme alkyle et alkyle cyclique ayant la signification donnée ci-dessus.

Par « aromatique », on entend un groupe mono- ou polycyclique aromatique, de préférence mono- ou bicyclique, ayant un nombre d'atomes de carbone entre 5 et 20. Lorsque le groupe est polycyclique, c'est-à-dire qu'il comprend plus d'un noyau cyclique, les noyaux cycliques peuvent avantageusement être condensés deux à deux ou rattachés deux à deux par des liaisons σ. Le groupe aromatique selon l'invention peut contenir un hétéroélément tel l'azote, l'oxygène ou le soufre.
Le terme ligand selon la présente invention est indifféremment utilisé pour signifier une ou plusieurs des formes limites répondant à la formule 1a), 1b) ou 1c) utilisée(s) pour former la composition selon l'invention.

Les deux groupements (R^{1a} et R^{1b}) peuvent être identiques ou différents entre eux. Ces deux groupements R^{1a} et R^{1b} peuvent également être liés entre eux. Dans un tel cas, les deux groupements R^{1a} et R^{1b} peuvent correspondre à des groupements tels que le bis-phényle, le bis-naphthyle.

Les ligands selon l'invention peuvent être préparés par une réaction de condensation d'un sulfonamide, par exemple le para-n-butylphényl-sulfonamide et d'un halogénure de phosphine, tel que Ph₂PCl, en présence d'une base de Brönsted telle que la triéthylamine par exemple, dans un solvant. En solution, ces ligands peuvent (co)exister sous les trois formes 1a), 1b) ou 1c) décrites ci-dessus.

La composition selon l'invention peut également comprendre une base de Lewis additionnelle. Au sens de la présente invention, on entend par « base de Lewis », toute entité chimique dont un constituant possède un doublet ou plus d'électrons libres ou non liants. Les bases de Lewis selon l'invention correspondent en particulier à tout ligand comprenant un atome d'oxygène, d'azote ou de phosphore possédant un doublet d'électrons libres ou non liants, ou une double liaison π capable de former avec le nickel une coordination de type η².

La base de Lewis additionnelle de la composition selon l'invention peut être une phosphine de type P(A¹R'^{1a})(A'¹R'^{1b})(A"¹R'^{1c}) ou une phosphinamine de type (R'^{1a}A')(R'^{1b}A'¹)P-NH(R'²) ou (R'^{1a}A¹)(R'^{1b}A'¹)P-NH-S(O)₂(R'²), dans lesquelles :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹, soit R'^{1a}, R'^{1b} et R'^{1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

Selon l'invention, les groupements R¹, soit R^{1a} et R^{1b}, identiques ou différents, liés ou non entre eux, et les groupements R'¹, R'^{1a}, R'^{1b} et R'^{1c}, identiques ou différents, liés ou non entre eux, sont indépendamment choisis parmi les groupements alkyles comprenant 1 à 15 atomes de carbones, les groupements aromatique comprenant 5 à 20 atomes de carbone; substitués ou non, contenant des hétéroéléments ou non.

De préférence, les groupements R¹, soit R^{1a} et R^{1b}, identiques ou différents, liés ou non entre eux, et les groupements R'¹, soit R'^{1a}, R'^{1b} et R'^{1c}, identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non. Dans le cas où les groupements soit R^{1a} et R^{1b} identiques ou différents, sont liés entre eux, ces groupements peuvent correspondre à des groupements tels que le bis-phényle, le bis-naphthyle. Dans le cas où les groupements R'¹ identiques ou différents sont liés entre eux, ces groupements peuvent correspondre à des groupements tels que le bis-phényle, le bis-naphthyle.

Selon l'invention, les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

De préférence, les groupements R² et les groupements R'², identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

De préférence le groupement R³ est soit un atome d'hydrogène, soit un groupement alkyle.

Les compositions selon l'invention peuvent être en présence ou non de solvant. On peut utiliser un solvant choisi parmi les solvants organiques et en particulier parmi les éthers, les alcools, les solvants chlorés et les hydrocarbures saturés, insaturés, aromatiques ou non, cycliques ou non. De préférence, le solvant est choisi parmi l'hexane, le cyclohexane, le méthylecyclohexane, l'heptane, le butane ou l'isobutane, les monooléfines ou dioléfines comportant de préférence 4 à 20 atomes de carbone, le cycloocta-1,5-diène, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où le solvant est un liquide ionique, il est avantageusement choisi parmi les liquides ioniques décrits dans les brevets US 6,951,831 B2 et FR 2895406 B1.

Lorsque le précurseur de nickel est de degré d'oxydation (0), ce dernier peut être choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange. Lesdits précurseurs de nickel peuvent éventuellement être complexés à des bases de Lewis.

Lorsque le précurseur de nickel est de degré d'oxydation (+II), ce dernier peut être choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange. Lesdits précurseurs de nickel peuvent éventuellement être complexés à des bases de Lewis.

Lorsque qu'un précurseur de nickel de degré d'oxydation (+II) est utilisé dans la composition, celui-ci est utilisé en présence d'un agent réducteur ou en présence d'une base de Brönsted.

On peut utiliser tout agent conduisant à la réduction du nickel connu de l'homme du métier. L'agent réducteur peut être choisi parmi NaBH₄, LiAlH₄, AlEt₃, Na, K, KC₈ et le dihydrogène.

On peut utiliser toute base de Brönsted connue de l'homme du métier. On entendra par « base de Brönsted » toute entité moléculaire ou espèce chimique correspondante capable d'accepter un proton, telle que par exemple la triéthylamine.

Selon l'invention, le rapport molaire entre le ou les ligand(s) répondant à la formule 1a), 1b) ou 1c) et le précurseur de nickel est avantageusement compris entre 0,05 et 10, de préférence entre 0,5 et 3.

Selon l'invention, le rapport molaire entre la base de Lewis et le précurseur de nickel est avantageusement compris entre 0,05 et 10, de préférence entre 0,5 et 3.

Une liste non exhaustive de ligands pouvant convenir à la préparation des compositions selon l'invention est représentée ci-dessous. Les ligands sont représentés sous leurs formes limites 1a) et 1b).

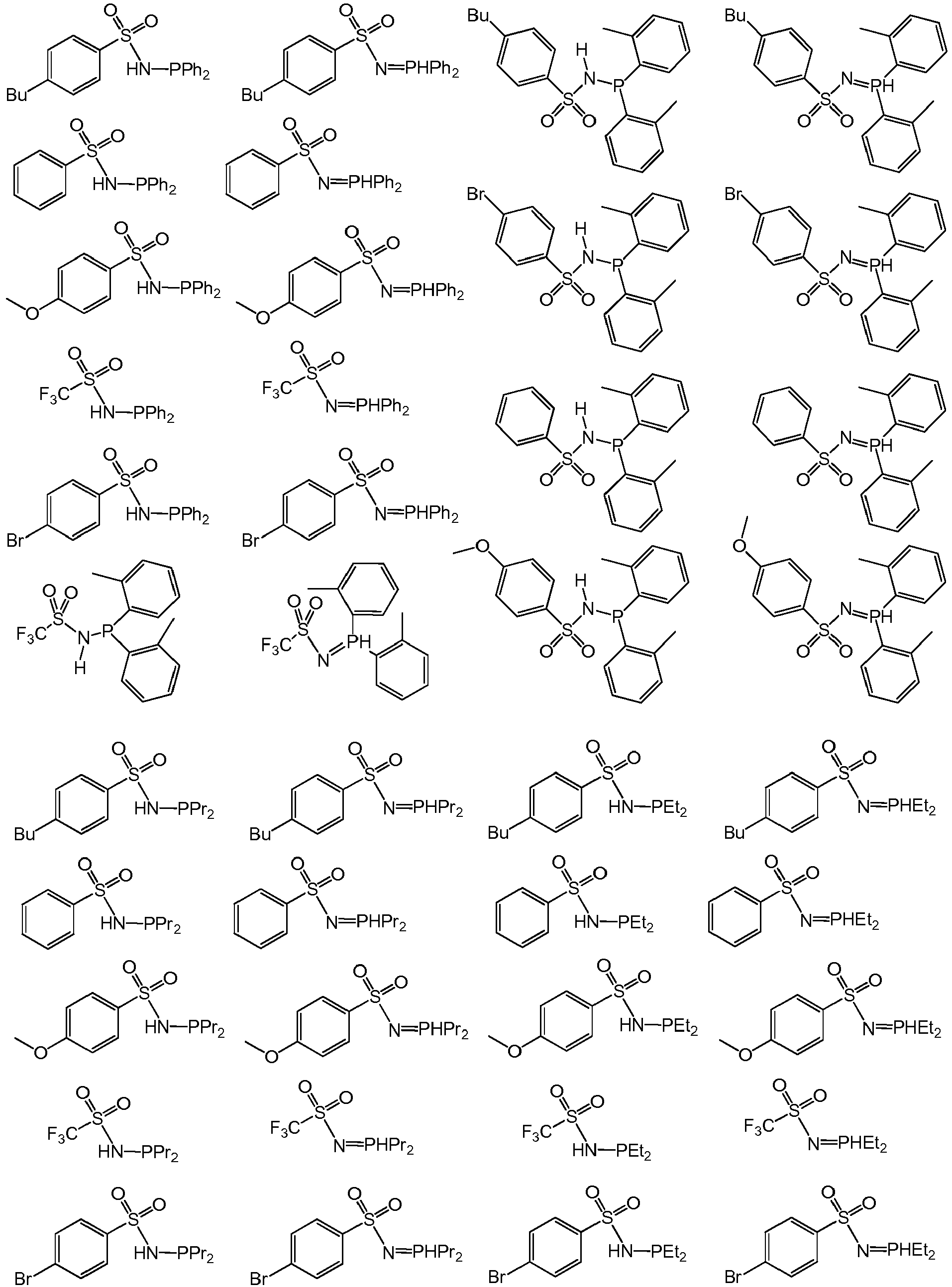

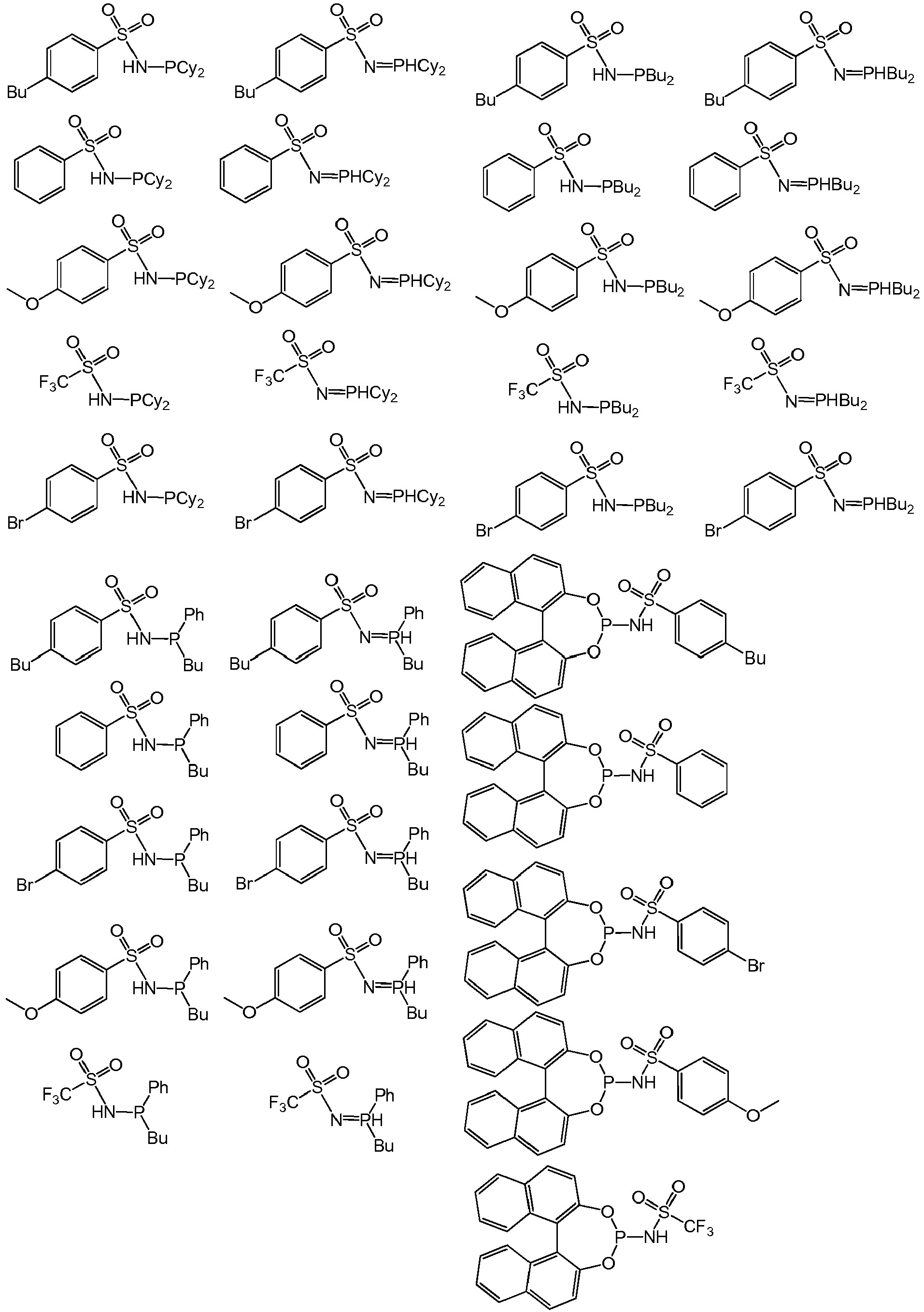

### Utilisation de la composition selon l'invention

Les compositions selon l'invention peuvent être utilisées comme catalyseur dans une réaction de transformation chimique, telle que la réaction d'hydrogénation, d'hydroformylation, de couplage croisé ou d'oligomerisation des oléfines. En particulier, ces compositions sont utilisées dans un procédé d'oligomerisation d'une charge d'oléfines ayant avantageusement 2 à 10 atomes de carbone.

De préférence, le procédé d'oligomerisation est un procédé de dimérisation de l'éthylène en butene-1.

Les compositions selon l'invention peuvent comprendre en outre un composé appelé agent activateur. Ledit agent activateur est avantageusement choisi dans le groupe formé par les composés tris(hydrocarbyl)aluminium, les composés chlorés ou bromés d'hydrocarbylaluminium, les halogénures d'aluminium, les aluminoxanes, les composés organo-borés, les composés organiques susceptibles de donner ou de capter un proton, pris seuls ou en mélange.

Les tris(hydrocarbyl)aluminium, les composés chlorés ou bromés d'hydrocarbylaluminium et les halogénures d'aluminium répondent de préférence à la formule générale AlₓR_{y}W_{z} dans laquelle R représente un radical hydrocarboné monovalent contenant par exemple jusqu'à 12 atomes de carbone tel que alkyle, aryle, aralkyle, alkaryle ou cycloalkyle, W représente un atome d'halogène choisi par exemple parmi le chlore et le brome, W étant de préférence un atome de chlore, x prend une valeur de 1 à 2, y et z prennent une valeur de 0 à 3. Comme exemples de tels composés, on peut mentionner le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), le triméthylaluminium, le tributylaluminium, le tri-n-octylaluminium et le triéthylaluminium (AlEt₃).

Dans le cas où ledit agent activateur est choisi parmi les aluminoxanes, ledit agent activateur est avantageusement choisi parmi le méthylaluminoxane (MAO), l'éthylaluminoxane et les méthylaluminoxanes modifiés (MMAO). Ces agents activateurs peuvent être utilisés seuls ou en mélange.

De préférence, ledit agent activateur est choisi parmi le dichloroéthylaluminium (EtAlCl₂) et le méthylaluminoxane (MAO).

Dans le cas où ledit agent activateur est choisi parmi les composés organoborés, ledit agent activateur est de préférence choisi parmi les acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés et les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Dans le cas où ledit agent activateur est choisi parmi les composés organiques susceptibles de donner un proton, ledit agent activateur est de préférence choisi parmi les acides de formule HY dans lequel Y représente un anion.

Dans le cas où ledit agent activateur est choisi parmi les composés organiques susceptibles de capter un proton, ledit agent activateur est de préférence choisi parmi les bases de Brönsted.

Le solvant du procédé d'oligomérisation peut être choisi parmi les solvants organiques et de préférence parmi les éthers, les alcools, les solvants chlorés et les hydrocarbures saturés, insaturés, aromatiques ou non, cycliques ou non. En particulier, ledit solvant est choisi parmi l'hexane, le cyclohexane, le méthylecyclohexane, l'heptane, le butane ou l'isobutane, les monooléfines ou dioléfines comportant de préférence 4 à 20 atomes de carbone, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où ledit solvant de réaction est un liquide ionique, il est avantageusement choisi parmi les liquides ioniques décrits dans les brevets US 6,951,831 B2 et FR 2895406 B1.

L'oligomérisation est définie comme la transformation d'une unité monomère en un composé ou mélange de composés de formule générale CₚH₂ₚ avec 4 ≤ p ≤ 80, de préférence avec 4 ≤ p ≤ 50, de manière préférée avec 4 ≤ p ≤ 26 et de manière plus préférée avec 4 ≤ p ≤ 14.

Les oléfines utilisées dans le procédé d'oligomérisation sont des oléfines comportant de 2 à 10 atomes de carbone. De préférence, lesdites oléfines sont choisies parmi l'éthylène, le propylène, les n-butènes et les n-pentènes, seules ou en mélange, pures ou diluées.

Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur.

De manière préférée, l'oléfine utilisée dans le procédé d'oligomérisation est l'éthylène.

Lesdites oléfines peuvent venir de ressources non fossiles telles que la biomasse. Par exemple, les oléfines utilisées dans le procédé d'oligomérisation selon l'invention peuvent être produites à partir d'alcools, et en particulier par déshydratation des alcools.

La concentration du nickel dans la solution catalytique est avantageusement comprise entre 1.10⁻⁸ et 1 mol/L, et de préférence entre 1.10⁻⁶ et 1.10⁻² mol/L.

Le procédé d'oligomérisation opère avantageusement à une pression totale comprise entre la pression atmosphérique et 20 MPa, de préférence entre 0,1 et 8 MPa, et à une température comprise entre -40 et +250°C, de préférence entre -20°C et 150°C.

La chaleur engendrée par la réaction peut être éliminée par tous les moyens connus de l'homme du métier.

Le procédé d'oligomérisation peut être conduit en système fermé, en système semi-ouvert ou en continu, avec un ou plusieurs étages de réaction. Une vigoureuse agitation est avantageusement mise en oeuvre pour assurer un bon contact entre le ou les réactifs et le système catalytique.

Le procédé d'oligomérisation peut être mis en oeuvre en discontinu. Dans ce cas, un volume choisi de la solution comprenant la composition selon l'invention est introduit dans un réacteur muni des dispositifs habituels d'agitation, de chauffage et de refroidissement.

Le procédé d'oligomerisation peut également être mis en oeuvre en continu. Dans ce cas, la solution comprenant la composition selon l'invention est injectée en même temps que l'oléfine dans un réacteur agité par les moyens mécaniques classiques ou par une recirculation extérieure, et maintenue à la température souhaitée.

La composition catalytique est détruite par tout moyen habituel connu par l'homme du métier, puis les produits de la réaction ainsi que le solvant sont séparés, par exemple par distillation. L'oléfine qui n'a pas été transformée peut être recyclée dans le réacteur.

Le procédé selon l'invention peut être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, la composition catalytique peut être désactivée, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools, comme composants pour l'industrie chimique, pharmaceutique ou la parfumerie et/ou comme charges dans un procédé de métathèse pour la synthèse de propylène par exemple.

Les exemples suivants illustrent l'invention sans en limiter la portée.

### Exemple 1 :

### Synthèse du ligand L1

La synthèse du ligand **L1** a été réalisée selon la méthode décrite dans la littérature : F. G. Terrade, Eur. J. Inorg. Chem. 2014, 1826-1835**.**

### Préparation des compositions selon l'invention

### Préparation de la composition C1

Le ligand **L1** (4-nBu-C₆H₄-SO₂-NH-PPh₂, 22 mg, 40 µmol, 1 eq), la triméthylphosphine (solution 1 M dans le toluène, 88 µL, 40 µmol, 1 eq.) et du Ni(COD)₂ (31.8 mg, 40 µmol, 1 eq) sont mis en suspension dans 10 mL de toluène et agités pendant 30 min. Puis, 5 mL de cette solution (soit 20 µmol de Ni) sont prélevés et injectés dans le réacteur.

### Préparation de la composition C2

Le ligand **L1** (4-nBu-C₆H₄-SO₂-NH-PPh₂, 31.8 mg, 80 µmol, 2 eq.) et du Ni(COD)₂ (11 mg, 40 µmol, 1 eq.) sont dissous dans du toluène (10 mL) et le mélange est agité pendant 30 min. Deux expériences sont menées en parallèle avec deux solutions de concentrations différentes de ladite composition C2, une solution à 20 µmol de Ni et une autre à 100 µmol de Ni sont évaluées dans un réacteur.

### Exemple 2 : oligomérisation de l'éthylène

La réaction d'oligomérisation de l'éthylène a été évaluée avec les compositions **C1** et **C2**. Les résultats obtenus sont reportés dans le tableau 1.

Le réacteur de 250 mL est séché sous vide à 130°C pendant 2 heures puis pressurisé avec 0,5 MPa d'éthylène. La température est abaissée à 20°C, puis la surpression d'éthylène est évacuée pour atteindre 0,1 MPa. Le solvant est ajouté (45 mL de toluène), et la consigne de température interne est fixée (40°C ou 80°C). Une fois la température interne stabilisée, une partie de la composition catalytique **C1** ou la composition catalytique **C2** est introduite (20 µmol de Ni ou 100 µmol de Ni). Ensuite le réacteur est pressurisé avec de l'éthylène à 3 MPa. L'agitation (1000 tr/min) est mise en route (t=0). Après le temps de réaction défini, le mélange est refroidi à 30°C sous agitation, le réacteur est dépressurisé et les phases liquide et gaz sont analysées par chromatographie en phase gazeuse (GC).

La productivité (g_{oligo}/(g_{Ni·}h) est exprimée en masse d'oligomères produits (en gramme) par masse de nickel mis en oeuvre et par heure.

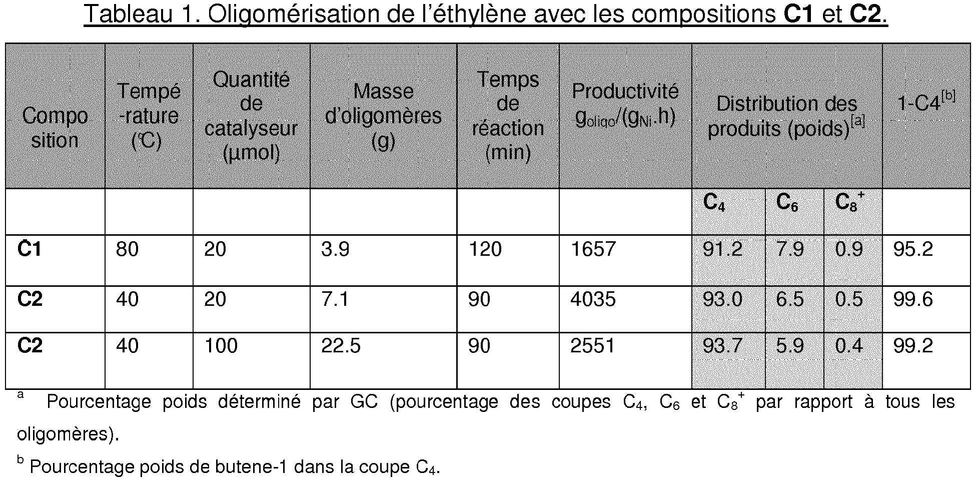

Les exemples ci-dessus démontrent que les compositions selon l'invention présentent une bonne activité pour l'oligomérisation de l'éthylène.

## Revendications

1. Composition comprenant :
- au moins un précurseur de nickel de degré d'oxydation (0) ou (+II),
- au moins un ligand répondant à la formule 1 a), 1 b) ou 1 c) 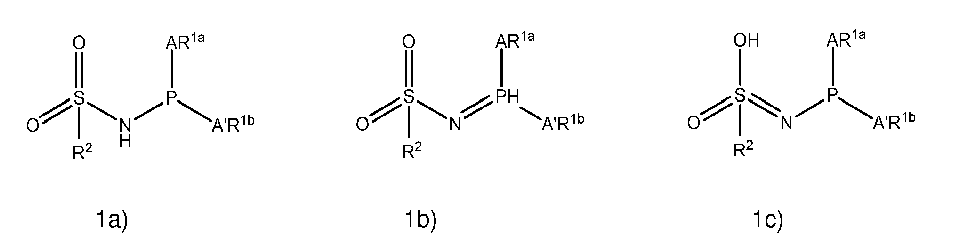 dans laquelle
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments,
avec la condition que lorsque qu'un précurseur de nickel de degré d'oxydation (+II) est utilisé dans la composition, celui-ci est utilisé en présence d'un agent réducteur ou en présence d'une base de Brönsted.

2. Composition selon la revendication 1 comprenant une base de Lewis additionnelle.

3. Composition selon la revendication 2 dans laquelle la base de Lewis additionnelle est une phosphine de type P(A¹R'^{1a})(A'¹R'^{1b})(A"¹R'^{1c}) ou une phosphinamine de type (R'^{1a}A¹)(R'^{1b}A'¹)P-NH(R'²) ou (R'^{1a}A¹)(R'^{1b}A'¹)P-NH-S(O)₂R'²), dans lesquelles :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹, soit R'^{1a}, R'^{1b} et R'^{1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

4. Composition selon l'une des revendications 1 à 3 dans laquelle les groupements R¹, soit R^{1a} et R^{1b} étant identiques ou différents, liés ou non entre eux, et les groupements R'¹, soit R^{1a}, R'^{1b} et R'^{1c} étant identiques ou différents, liés ou non entre eux, sont indépendamment choisis parmi les groupements alkyles comprenant 1 à 15 atomes de carbones, les groupements aromatique comprenant 5 à 20 atomes de carbone; substitués ou non, contenant des hétéroéléments ou non.

5. Composition selon l'une des revendications 1 à 4 dans laquelle , les groupements R¹, soit R^{1a} et R^{1b} étant identiques ou différents, liés ou non entre eux, et les groupements R'¹, soit R^{1a}, R'^{1b} et R'^{1c} étant identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non.

6. Composition selon l'une des revendications 1 à 5 dans laquelle les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

7. Composition selon l'une des revendications 1 à 6 dans laquelle les groupements R² et les groupements R'², identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

8. Composition selon l'une des revendications 1 à 7 dans laquelle lorsque le précurseur de nickel est de degré d'oxydation (0), ce dernier peut être choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange.

9. Composition selon l'une des revendications 1 à 7 dans laquelle le précurseur de nickel de degré d'oxydation (+II) est choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange.

10. Composition selon l'une des revendications précédentes comprenant en outre un agent activateur choisi dans le groupe formé par les composés tris(hydrocarbyl)aluminium, les composés chlorés ou bromés d'hydrocarbylaluminium, les aluminoxanes, les composés organo-borés, les composés organiques susceptibles de donner ou capter un proton, pris seuls ou en mélange.

11. Composition selon l'une des revendications précédentes dans laquelle le rapport molaire entre le ligand et le précurseur de nickel est compris entre 0,05 et 10.

12. Utilisation d'une composition selon l'une des revendications 1 à 11 en tant que catalyseur.

13. Procédé d'oligomérisation d'une charge d'oléfines comprenant la mise en contact de ladite charge avec une composition selon l'une des revendications 1 à 11.

14. Procédé selon la revendication 13 dans lequel la charge comprend des oléfines ayant un nombre d'atomes de carbone compris entre 2 et 10.

15. Procédé selon la revendications 13 ou 14 dans lequel la réaction est une réaction d'oligomérisation de l'éthylène.
